# EUROPEAN PATENT SPECIFICATION

(11) **EP 3 445 313 B1**
(45) Date of publication and mention of the grant of the patent: **18.12.2019**
(21) Application number: 17715693.2
(22) Date of filing: 04.04.2017
(51) Int. Cl.: A61K 8/06, A61K 8/44, A61Q 19/10

(54) **NOVEL NANOEMULSIONS COMPRISING N-ACYL AMINO ACID SALT AND PROCESS FOR MAKING**
NEUARTIGE NANOEMULSIONEN ENTHALTEND EIN N-ACYL AMINOSÄURESALZ UND HERSTELLUNGSVERFAHREN
NOUVELLES NANOÉMULSIONS COMPRENANT UN SEL DE N-ACYL ACIDE AMINÉ ET PROCÉDÉ DE FABRICATION

(30) Priority: 21.04.2016 EP 16166486
(43) Date of publication of application: 27.02.2019
(73) Proprietor: Unilever PLC, London, Greater London EC4Y 0DY (GB); Unilever N.V., 3013 AL Rotterdam (NL)
(72) Inventor: QUAN, Congling, Trumbull, CT 06611 TEST (US); LANG, David, John, Trumbull, CT 06611 TEST (US)
(74) Representative: Fijnvandraat, Arnoldus
(86) International application number: PCT/EP2017/057963
(87) International publication number: WO 2017/182262

(56) References cited:
- CN-A- 105 287 235
- KR-A- 20110 072 022
- KR-A- 20140 066 362
- US-A1- 2003 077 299

## Description

### Field of the invention

The present invention relates to novel oil-in-water (o/w) nanoemulsions and to process for making. The nanoemulsions are based on the combination of (1) an internal oil phase having triglyceride oils and/or petrolatum preferably in amounts of greater than 40% to 75%, preferably 41 to 75% or 41 to 70% by wt. total nanoemulsion; and (2) an external aqueous phase containing surfactants which are salts of N-acyl derivatives of dicarboxylic amino acids (e.g., aspartic acid, glutamic acid). The process is used to make compositions having droplet size of 20 to 500 nanometers (nm), preferably 20 to 400 nm.

The invention is concerned with the provision of such triglyceride oils and petrolatum (benefit agents delivered from nanoemulsion) in small droplets (e.g., 500 nanometers or less; for compositions comprising triglycerides, preferably droplet size is 225 nm or less, e.g., 20 to 225 nm; for those with petrolatum, droplet size is preferably 20 to 400 nm), which are more aesthetically pleasing than compositions in which benefit agents are delivered in the form of larger oil droplets. The nanoemulsions further provide high deposition of the triglyceride oil and/or petrolatum when being incorporated in personal cleansing compositions. Further, surprisingly, excellent lather performance of personal cleansing compositions is found when these benefit agents are present in the form of small droplets as noted . Typically, the triglyceride oil and petrolatum benefit agents tend to depress lather speed and volume when in the form of droplets of a few microns.

The invention further contemplates personal care cleansing compositions consisting essentially of the novel nanoemulsions of the invention in combination with cationic polymers to enhance deposition of benefit agents even further. Thus, surprisingly the nanoemulsions function as stand-alone compositions providing excellent benefit agent deposition and not just as ingredients which can be incorporated into more complex personal cleansing compositions.

### Background of the invention

Skin moisturizing oils (including triglyceride oils and petrolatum benefit agents noted above) are often delivered from personal cleansing compositions (e.g., shower gels, facial and hand cleansers designed to cleanse and moisturize skin) in the form of large oil drops (e.g., 50 to 200 microns or greater).

U.S. Patent Nos. 5,584,293 and 6,066,608, both to Glenn, Jr., for example, disclose a moisturizing liquid personal cleansing emulsion with at least 10% lipophilic skin moisturizing agent droplets having a diameter of greater than 200 microns.

U.S. Patent No. 8,772,212 to Restrepo et al. discloses an isotropic cleansing composition containing high level of petrolatum; greater than 50% by volume of the petrolatum particles have a diameter greater than 50, 100, 150 or 200 microns.

Compositions containing large oil drops need to be well structured so they can suspend the large droplets (using, for example, stabilizers). U.S. Patent Nos. 5,854,293 and 6,066,608, for example, utilize stabilizers selected from crystalline, hydroxyl-containing stabilizers, polymeric thickeners, C10-C18 diesters, amorphous silica or smectite clay. Special blending processes are typically needed to prepare such compositions. For example, compositions must be prepared under low shear to prevent oil droplet size reduction (see U.S .Patent No. 8,772,212). Although they provide enhanced delivery of benefit agents, these products are generally considered to be less aesthetically appealing to the consumer due to the presence of large oil droplets.

Another method of enhancing the delivery of a benefit agent (e.g., silicone) to the skin, for example, is through the use of cationic hydrophilic polymers such as, for example, hydroxypropyltrimethylammonium derivative of guar gum, sold under the name JAGUAR® C-13-S (see U.S. Patent No. 5,500,152 to Helliwell). In this reference, silicone oil is a preformed emulsion with oil droplet size ranging from 0.1-1 micron (µm), with a mean particle size of 0.4 µm (there is no mention whether this refers to number average or volume average diameter of droplets). This kind of product tends to be smooth and aesthetically appealing. However, nourishing vegetable oils (triglyceride oils) and highly occlusive skin protectants, such as petrolatum, are typically preferred moisturizers from a cleansing composition. It is also noted that fully formulated shower gel compositions comprise a completely different surfactant system (isethionate and betaine) than the nonionic surfactant lauryl alcohol ethoxylates used in the nanoemulsions of U.S. Patent No. 5,500,152 to Helliwell.

One challenge facing cleansing compositions that are rich in moisturizing oils is that large amount of oils (especially at levels such as those of our invention when used at levels of greater than 40% of nanoemulsion) tend to depress the lather speed and volume.

It is therefore desirable to prepare a personal cleansing composition consisting of triglyceride oils and/or petrolatum nanoemulsion, which is aesthetically appealing, high in deposition of these moisturizing oils, and which maintains high lather performance.

In the subject invention, applicants provide novel nanoemulsions for delivery of triglyceride oils and petrolatum as small (20 to 500 preferably 50 to 400 nm) volume average diameter droplets. Further, unexpectedly, high lather performance is maintained. For triglycerides preferred levels are 41 to 75% of nanoemulsion and preferred size is 20 to 225 nm. For petrolatum same levels are preferred, but with droplet size of 20 to 400 nm. Homogenization is typically done at 4000 to 6000 psi (e.g., 5000 psi)
Nanoemulsions of the invention comprise (1) an oil phase containing benefit agent droplets selected from the group consisting of triglyceride oil, petrolatum and mixtures thereof; and (2) an aqueous phase comprising one or more surfactants which are salts of N-acyl derivatives of dicarboxylic amino acids; specifically, these surfactants may be selected from (a) salt of acylglutamic acid or salt of acylaspartic acid with defined N-acyl groups, or (b) mixtures of the salts acylglutamic and acylaspartic acid.

The specific N-acyl derivatives of dicarboxylic amino acids (e.g., aspartic acid and glutamic acid salts) comprise 50% or greater, preferably 60% or greater of all surfactants present in the aqueous phase of the nanoemulson composition.

Both U.S. Patent Nos. 8,834,903 and U.S. Patent No. 6,541,018 to Simonnet et al. disclose nanoemulsion compositions in which acylglutamate is mentioned as possible surfactant (e.g., U.S. 8,834,903 at column 4, lines 27-31). However, it is disclosed as one of many possible surfactants and, if used, the surfactants are used as "additional" components, e.g., as co-surfactant (column 4, line 53). In the examples, the glutamate is never used at levels greater than 0.5% (10% by wt. of total surfactant). The exemplified glutamate is also a salt of N-stearoyl-glutamic acid. This has C₁₈ chain length and provides poor lather in a cleansing composition.

In U.S. Patent No. 6,541,018, the internal phase oils are primarily lower molecular weight ester oils (MW less than 400). The lower MW ester oil impacts viscosity and foam of cleansing compositions. The triglycerides and the petrolatum (having melting point from 30° to 60°C) of our invention show less impact on viscosity and foam.

It is further noted that nanoemulsions disclosed in US 8,834,903 and US 6,541,018 have an internal phase where concentration of oil is no higher than 40% of the emulsion. While the concentration of oils of the subject invention may range from 20% to 75% by wt. of total nanoemulsion, preferred ranges are 41 to 75%, preferably 41% to 70% or 42 to 65%. The specific surfactants and oils of the invention can be used to form nanoemulsions at these higher oil concentration ranges and indeed, remarkably, at these higher, preferred oil concentration ranges, applicants have unexpectedly found that volume average diameters of oil droplets is lower than when the oil level is at lower oil concentration ranges, even at the same processing pressure and same surfactant and oil ratio. This is beneficial not only because it consumes less energy to prepare nanoemulsions of smaller droplets, but it also improves the yield of nano oil droplets. Even further, the low volume average diameter droplet nanoemulsions are formed at pressures lower than those used to form the nanoemulsions of the aforementioned references.

It is also noted that, when the size of oil globules is defined in the Simonnet patents (see column 2, line 64 of US 8,834,903), it is defined by number average. Since number average is the simple averaging of size of all particles (e.g., 1µm droplet plus 99µm droplet average to about 50µm) they do not account for volume average diameter of droplet (e.g., volume average diameter of 1µ droplet and 99µ droplet is much closer to 99µm). Thus, it is not clear that these references disclose the same low volume average drops as disclosed in our invention.

U.S. Publication No. 2014/0113852 relates to a method for producing concentrated water-continuous emulsions containing lipophilic compounds in a very fine droplet dispersed phase. This is done using a Controlled Deformation Dynamic Mixer (CDDM) or Cavity Transfer Mixer (CTM). Formation of mild personal cleansing products using specific amino acid surfactants while providing aesthetically pleasing products containing triglycerides and/or petrolatum which maintain excellent lather is not recognized or disclosed.

CN 105287235 discloses nanoemulsions used in a whitening mask. Although glutamate is noted as possible surfactant, it is used at levels less than 40% of surfactant system.

KR 101419602 discloses phytosterol based drug carrier and methods to prepare. While nanoemulsions can be made, the amount of triglyceride, if any, is well below 40%.

The unique nanoemulsions of the present invention contain small oil droplets (500 or less, preferably 400 or less) which are aesthetically pleasing, efficiently deliver the benefit agent triglycerides oils or petrolatum, and maintain excellent lather when being incorporated in the personal cleansing compositions. Further, the specific surfactants used, including the chain length of fatty acids used to form the surfactant, provide excellent, "mild" cleansing and ensure foam maintenance when the nanoemulsions are used in personal cleansing products. Preferably, the triglycerides and/or oils comprise 40 to 75% or 41 to 75% of or 42 to 70% by wt. of nanoemulsion.

With regard to mildness of surfactant, applicants note "Effect of surfactant mixtures on irritant contact dermatitis potential in man: sodium lauroyl glutamate and sodium lauryl sulphate" by C.H. Lee et al (Contact Dermatitis, Volume 30, Issue 4, pages 205-209, April 1994); and M. Sugar and R. Schmucker "Reduction of Skin's Surfactant Adsorption: An Effective Way To Improve Mildness And Performance of Bath Care Products"(XXI IFSCC International Congress 2000, Berlin-Proceedings), wherein is disclosed that sodium lauroyl glutamate and sodium cocyl glutamate (e.g., N-acyl derivatives of dicarboxylic acid) are mild surfactants and their utilization can decrease irritation potential in Sodium lauryl sulphate and SLES.

### Brief Description of the Invention

Specifically, in one form the present invention relates to nanoemulsion compositions comprising:
a) an internal phase comprising 40 to 75% by wt., preferably 41 to 75% or 41 to 70% or 42 to 65% by wt. of total nanoemulsion of oil selected from the group consisting of triglyceride oils, petrolatum and mixtures thereof, wherein the melting point of the petrolatum oil forming the droplets is 30 to 60°C; and
b) an external aqueous phase comprising 2 to 15% by wt. (as active) of total nanoemulsion of a surfactant or surfactants which are N-acyl derivatives of di-carboxylic amino acid, preferably, said surfactant or surfactants is selected from the group consisting of
   (i) salt of acylglutamic acid, wherein greater than 65% (e.g., 65 to 100%, preferably 65 to 90%) of the acyl group has chain length of C₁₄ or less;
   (ii) salt of acylaspartic acid, wherein greater than 65% of the acyl group (e.g., 65 to 100%, preferably 65 to 90%) has chain length C₁₄ or less; and
   (iii) mixtures thereof;
   wherein the surfactant of (b) comprises 50% or greater, preferably 60% or greater, preferably 65 to 100% of all surfactants present in the aqueous phase of the nanoemulsion;
   wherein the volume average diameter of the oil droplets of (a) is 20 to 500 nanometers.

In one form the internal phase comprises triglyceride oils and volume average diameter of oil droplets is 20 to 225 nm, preferably 25 to 220 nm.

In another form, the internal phase is petrolatum oil and volume average diameter of droplets is 20 to 400 nm, preferably 25 to 350.

It should be understood that the claims are directed to the composition. That is the claims are intended to cover the salts of N-acyl derivatives of dicarboxylic acids, for example, whether formed by us or bought as a prepared surfactant product (as would occur in the vast majority of all cases).

In one form, the nanoemulsion contains 40 to 75% by wt., preferably 41 to 70% by wt. nanoemulsion of oils and the volume average diameter of the droplets is 20 to 400 or 50 to 300 nanometers. As noted, when internal phase is triglyceride, preferably droplet size is 20 to 225 nm. When the nanoemulsion contains 20 to less than 40% by wt. or less than 41% by wt. nanoemulsion oils, applicants have found that this volume average diameter is typically larger, even when using similar preparation methods.

The nanoemulsions are typically prepared by mixing the oil phase and the aqueous phase using the conventional rotor/stator high shear devices and further processed via a homogenizer at a process pressure of 7000 pounds per square inch (psi) or less, preferably 6000 psi or less; preferably 5000 psi or less.

Because greater than 65% of the acyl group in the surfactant has chain length of C₁₄ or less, the nanoemulsion composition, once formed, provides several advantages. For example, the nanoemulsion composition can be readily incorporated into personal cleanser liquids which are structured by micelles or are lamellar structured. Further, the predominantly shorter chain N-acyl groups (relative to longer chain C₁₆ and C₁₈, for example) on the surfactant enable good lather formation in the cleanser liquids.

Thus, the novel nanoemulsions are sensorially pleasing (due to small droplet size), provide efficient oil deposition, provide superior stability (again because of smaller droplet size), and are ideally suited (because of chain length selection) for use in personal cleansing liquids while providing excellent lather.

The invention also contemplates compositions which consist essentially of nanoemulsion compositions and cationic polymers. That is, after preparing the nanoemulsion compositions, they can be combined with small amounts of cationic polymer (some dispersing agent is typically used to combine the two) to form a stand-alone personal care cleansing composition which can be packaged and distributed as is. This results in savings as a result of the use of fewer ingredients and less complex formulating, while still delivering excellent cleansing and benefit agent deposition benefit.

In one form the invention comprises a process for making any of the nanoemulsion compositions noted wherein said process comprises:
1) heating aqueous phase to about 55 to about 75°C;
2) heating oil phase to about 55 to about 75°C or until oil is molten;
3) adding oil phase to aqueous phase to form a coarse emulsion with a conventional rotator/stator high shear device at speed of 1000 to 6000 revolutions per minute (rpm) ;
4) pumping the coarse emulsion once or multiple times through a homogenizer at a process pressure of 7000 psi or less, preferably 6000 psi or less; preferably 5000 psi or less; and
5) cooling emulsion to room temperature.

In step 3), alternatively, the coarse emulsion may be formed using a homogenizer operating at pressure of 200 to 500 psi.

### Detailed description of the invention

Except in the examples, or where otherwise explicitly indicated, all numbers in this description indicating amounts of material or conditions of reaction, physical properties of materials and/or use are to be understood as modified by the word "about." All amounts are by weight of the final composition, unless otherwise specified.

It should be noted that in specifying any range of concentration or amount, any particular upper concentration can be associated with any particular lower concentration or amount.

For the avoidance of doubt, the word "comprising" is intended to mean "including" but not necessarily "consisting of" or "composed of." In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

The present invention provides novel nanoemulsions containing a specific selection of benefit agents and surfactants and process for making. The nanoemulsions can be prepared using lower processing pressure of 7000 psi or less. The novel nanoemulsions are ideally suited for use in liquid cleansing compositions.

Specifically, the N-acyl derivatives of dicarboxylic amino acid surfactants (e.g., acylglutamate and/or acylaspartate surfactants) have greater than 65%, preferably greater than 75%, preferably greater than 80% of C₁₄ or less acyl chain (preferably they have greater than 75% acyl chain which are C₁₂, C₁₄ and mixtures thereof). The chosen surfactants provide multiple advantages when final nanoemulsions are mixed into fully formulated liquid personal cleansing compositions. First, the glutamate and aspartate surfactants are known to be less irritating than harsher surfactants typically used such as sodium lauryl sulphates and sodium lauryl ether sulphaste (SLES). Also, as noted, the chain length is selected so the surfactants are suitable for use in structured personal cleansing liquids while providing minimal interference with such structuring. Further, the selected predominantly shorter chain lengths (relative to longer chain C₁₆ and C₁₈) ensure the surfactants will provide good foam.

The nanoemulsions also, quite unexpectedly, form droplets of small volume average diameter even when oil phase greater than 40% to 75%, e.g., 41 to 70% of the total nanoemulsion is used. Such droplets of small volume average size help provide more efficient deposition. For example, cationic polymers typically used in fully formulated liquid cleanser more readily deposit the smaller droplets than larger droplets. Large oil droplets also require stabilizers to suspend the large oil droplets. The small size oil droplets from the nanoemulsion, when incorporated into a cleansing liquid, also provide greater stability. Small droplets are also viewed as more aesthetically pleasing.

The nanoemulsions of the invention are defined with more particularity below.

### Oil Phase

Oil phase of the nanoemulsions may be triglyceride oil or oils (animal and/or vegetable oils); petrolatum; or mixtures of one or more triglyceride oil and petrolatum when the oil phase comprises triglycerides, preferred droplet sizes are smaller (20 to 225 nm) than when it comprises petrolatum.

Examples of triglyceride oils which may be used include soybean oil, sunflower seed oil, coconut oil, rapeseed oil, palm oil, palm kernel oil, grape seed oils and fish oil. Soybean and sunflower seed oils are preferred triglycerides.

The oil phase may also be petrolatum. The petrolatum preferably has a melting point ranging from 30° to about 60°C. Examples of such petrolatum include Vaseline® Petrolatum Jelly from Unilever, White Petrolatum USP from Calumet Penreco, Petrolatum G2212 and White Protopet® 1S from Sonneborn.

The oil phase can range from 20% to 75% by wt., preferably 40 to 75% by wt. or 41 to 75 or 41 to 70% or 42 to 70% or 42 to 65% of the total nanoemulsion composition. It has been unexpectedly found that at preferred higher levels, that is, at level of greater than 40% to 75% by wt. total nanoemulsion, the volume average diameter of the droplets is smaller than when oil level is less than 40%. This is beneficial because, at the high oil levels, lower process pressure is needed to obtain desired oil droplet size, resulting in a higher yield of fine oil droplets while using low energy consumption. The preferred volume average diameter of the triglyceride oil or petrolatum droplets is 20 to 500 nm, preferably 50 to 400nm, most preferably 50 to 300nm. Upper level can be 250 or 200 or 175. For triglycerides, preferred size is 20 to 225 or 25 to 220. For petrolatum, preferred droplet size is 20 to 400 or 50 to 350 nm.

The choice of triglyceride oils and petrolatum helps impart nourishment, emolliency and occlusivity to skin when the triglyceride oils and/or petrolatum deposit onto skin after the skin is washed with fully formulated cleansing compositions into which the nanoemulsions of this invention have been incorporated.

In addition to the triglyceride oil and/or petrolatum, the oil phase may comprise oil soluble skin beneficial actives such as, for example, Vitamin A, Vitamin E, sun screen, fragrances, retinol palmitate, 12-hydroxystearic acid, conjugated linoleic acid; antibacterial agent, mosquito repellent, etc. at level of 0.01 to 2%.

Another ingredient which might be found in the oil phase is an oil phase stabilizer. For example, small amounts (0.01 to 2%, preferably 0.1-1% by wt. nanoemulsion) of antioxidant may be used. When the oil used is triglyceride, a preferred antioxidant which may be used is butylated hydroxytoluene (BHT). This is often used as a food grade antioxidant.

### Aqueous Phase

The aqueous phase contains salts of N-acyl derivatives of dicarboxylic amino acids as emulsifier (50% or greater, preferably 60% or greater of all surfactants in the aqueous phase). Preferred emulsifiers are acylglutamate and acylaspartate surfactants. Preferably, these are potassium and/or sodium salts of acylglutamate or acylaspartate, wherein greater than 65% of the acyl group has chain length C₁₄ or less, e.g., C₈ to C₁₄ (e.g., derived coconut fatty acid). The acyl groups preferably have greater than 75%, more preferably greater than 80% C₁₄ or less chain length. Preferably, greater than 75%, most preferably greater than 80% of the chain length are C₁₂, C₁₄ or mixtures thereof. These predominantly short chain acyl groups (relative to longer chain C16 and C18, for example) ensure that, when nanoemulsions of the invention are incorporated into fully formulated liquid cleansing compositions (especially structured liquid cleansing compositions), they do not interfere with structuring of the composition and help maintain or enhance foaming capacity.

Examples of glutamate surfactants include:
- sodium N-cocoyl-L -glutamate (e.g., Amisoft® CS-11 by Ajinomoto)
- sodium N-lauroyl-L- glutamate (e.g., Amisoft® LS-11 by Ajinomoto)
- sodium N-myristoyl-L-glutamate (Amisoft® MS-11 by Ajinomoto)
- potassium N-cocoyl_I-Glutamate (e.g., Amisoft® CK-11 by Ajinomoto)
- potassium N-myristoyl-L-glutamate (Amisoft® MK-11 by Ajinomoto).

Examples of aspartate surfactants include: Sodium N-lauroyl-L-aspartate from Zhejiang Taizhou TU-POLY Co., Ltd, Sodium Lauroyl Aspartate (AminoFoamer™ FLMS-P1 and AminoFoamer™ FLCS-S1 from Asahi Kasei Chemical Corporation).

Overall surfactants comprise 2 to 15% preferably 4 to 12% by wt. of total nanoemulsion. As indicated, the salts of N-acyl derivatives of dicarboxylic amino acid, preferably acylglutamate, acylaspartate or mixtures thereof are the principal surfactant of the nanoemulsion. They constitute 40% or greater, preferably 50% or greater of all surfactant in the aqueous phase. Preferably, they consitute greater than 60%, more preferably greater than 70%. They may of course be the only surfactant present.

Other mild cleansing surfactants can be used in the aqueous phase in addition to the primary surfactant. Anionic surfactants which may be used include sodium cocoyl isethionate, sodium lauroyl isethionate, and other amino acid based surfactants, such as sodium methyl cocoyl taurate, sodium lauroyl sarcosinate, sodium cocoyl sarcosinate, sodium cocoyl glycinate, sodium lauroyl glycinate and acylalaninate salt. Amphoterics such as coco betaine, cocamidopropyl betaine, sodium lauroamphoacetate, Lauramidopropyl hydroxysultaine and Cocamidopropyl hydroxysultaine can also be used. These co-surfactants are typically present at a level of less than 50%, preferably less than 40%, more preferably less than 30% of all surfactant aqueous phase.

Preferably, the aqueous phase may contain a preservative or preservatives. Typically, they are present at a level of 0.01 to 1.0%, preferably 0.1 to 0.5% by wt.

Nanoemulsions of the invention have volume average diameter (also used interchangeably in and with terms "volume mean diameter" or "volume average size") of 500 nm or less, preferably 20 nm to 400 nm. As indicated, preferred sizes varies for triglyceride versus petrolatum.

Nanoemulsions with droplet sizes of these ranges are obtained in the subject invention using relatively low pressure applied by high pressure sonolator or other types of high pressure homogenizer. Pressures used are 7000 psi or less, preferably 6000 psi or less, most preferable 5000 psi or less, e.g., 4000 to 5000 psi.

### Preparation of Nanoemulsion

Nanoemulsions are typically formed in a two stage process.

The first mixing stage is used to form a coarse emulsion. The oil phase and aqueous phase were heated up to 75°C separately such that each phase was clear and uniform; then the oil phase was mixed with the aqueous phase with intensive mixing. Intensive mixing can be accomplished via conventional means including mixing the materials in a stirred tank and passing the mixture through a rotor/stator mixer such as the Silverson® high shear in-line mixer or mixing them in the vessel with a high shear mixer such as the Scott® Turbon mixer. Alternatively, the coarse emulsion may be created by using a continuous high shear mixing device such as the standard Sonolator device produced by Sonic Corporation of Connecticut. These standard sonolators are normally operated at pressures of 200-500 psi to form coarse emulsion.

The second stage of the process is to pass the coarse emulsion through a high pressure homogenizer to form the nanoemulsion. Suitable high pressure homogenizers are the Nano DeBee, DeBee 2000, DeBee 3000, DeBee 4000 homogenizers of BEE International (Massachusetts, USA) and the High Pressure Sonolator device also produced by Sonic Corporation of Connecticut. These devices can be operated up to range 4000-5000 psi in order to produce nanoemulsions of less than 300 nm. For specific hydrophobic oils such as petrolatum, multiple passes through the Nano DeBEE may be required to reach the desired particle size.

In the examples, the following terms are defined as noted below:
Pass#: the number of times the emulsion passes through high pressure homogenizer
D[4, 3]: volume average diameter or volume mean diameter or volume average size
D[3, 2]: surface area mean diameter

The average diameters are determined by a Malvern Mastersizer.

Personal Cleanser Compositions

Nanoemulsions of the invention may be combined with cationic polymer to form stand-alone personal care product compositions.

Specifically, the nanoemulsion plus cationic polymer may be formed, packaged and sold as a final cleansing product without need of additional complex formulation. As such, there is potential savings due to formulation savings (fewer ingredients, less processing complexity) while simultaneously retaining the benefits of cleansing and benefit agent deposition.

Compositions comprise, 90% to 99%, preferably 92 to 98% or 93 to 97% nanoemulsion composition, and 1-10% of cationic polymer dispersing agent. Typically, cationic polymer is present at a level of 0.05 to 2%, preferably 0.05 to 1% or 0.05 to 0.5%, or 0.1 to 0.5% or 0.1 to 0.4% of the composition and dispersing agent is present at higher levels of 1 to 8%, preferably 2 to 6% of the composition.

Various cationic polymers may be used. Examples of cationic polymers include the cationic cellulose ethers described in U.S. Pat. Nos. 3,816,616 and 4,272,515 and which are available commercially from Union Carbide Corp. under the trade mark POLYMER JR. Other suitable materials are the cationic polygalactomannan gum derivatives described in U.S. Pat. No. 4,298,494 which are commercially available under the trade mark JAGUAR from Celanese-Stein Hall. An example of a suitable material is the hydroxypropyltrimethulammonium derivative of guar gum of the formula: 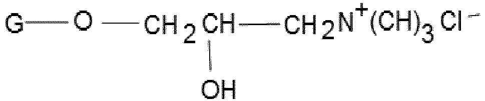 where G represents guar gum. Such a material is available under the name JAGUAR C-13-S. This material also has the CTFA designation Guar Hydroxypropyltrimonium Chloride. In JAGUAR C-13-S the degree of substitution of the cationic groups is about 0.13. Another possible material is that known as JAGUAR C-17 which is similar to JAGUAR C-13-S but has a higher degree of substitution of cationic groups of about 0.25-0.31. A further example of a guar derivative is the hydroxypropylated cationic guar derivative known as JAGUAR C-16 which as well as containing the above cationic quaternary ammonium groups also contain hydroxypropyl (---CH₂CH(OH)CH₃) substituent groups. In JAGUAR C-16 the degree of substitution of the cationic groups is 0.11-0.16 and the moles of substitution of hydroxypropyl groups is 0.8-1.1.

Other cationic polymers include cationic polyamide polymers such as the low molecular weight adipic acid/diethylene-triamine polyamide and the copolymers of vinylpyrrolidone and dimethylaminoethyl methacryate quaternized with dimethyl sulphase (Gafquat 755, GAF Corporation) described in U.S. Pat. No. 4,080,310; the graft cationic copolymer containing N-vinylpyrrolidone, dimethylaminoethyl methacrylate and polyethylene glycol described in U.S. Pat. No. 4,048,301; the mineral acid salts of the amino-alkyl esters of homo- and copolymers of unsaturated carboxylic acids having from 3 to 5 carbon atoms described in U.S. Pat. No. 4,009,256; and the polymers of etherified starch described in U.S. Pat. No. 3,186,911.

The high molecular weight cationic polymers are sold under the trade mark MERQUAT by Lubrizol. Representative ones are Merquat 100, a highly charged cationic dimethyldiallylammonium chloride homopolymer, and Merquat 550, a highly charged cationic copolymer prepared with dimethyldiallylammonium chloride and acrylamide.

As indicated, cationic polymer typically comprise 0.05 to about 2% or 0.05 to about 1% or 0.05 to 0.5%, preferably 0.1 to 0.5%, more preferably 0.1 to 0.4% of the composition.

The dispersing agent may be a small molecular weight alcohol such as polyethylene glycol or glycerol. It is typically present at a level at 0.9 to 9%, preferably 2 to 6%.

**Examples1-8:** Coarse emulsions were prepared in a one liter ESCO mixer equipped with a rotor/stator high shear device. The aqueous phase was added to the ESCO mixer and heated to about 55 to 75°C until aqueous phase was clear. The oil phase was combined and heated to about 55 to 75°C in a separate container. The oil phase was gradually added to the aqueous phase in the ESCO mixer under agitation and/or was homogenized by rotor/stator devices. When the addition of all oil phased was completed and the coarse emulsion was formed in the ESCO mixer, the coarse emulsion was transferred and passed through High Pressure homogenizer Nano DeBEE one or 2 times to arrive at the desired droplet size at a process pressure of 5000 psi. The pH value of nanoemulsions in the examples is typically between 5 and 6. The pH value can be adjusted to 5 to about 8 after nanoemulsion is formed.

Examples 1-2. Mono-sodium cocoyl glutamate was used as emulsifier for soybean oil nanoemulsions. Oil droplets were reduced to 243nm and 157 nm after passing Nano DeBEE once at 5000 psi with oil level of 35% and 50% respectively.

| | Example 1 | Example 2 (Comparative) |
|---|---|---|
| Ingredient | Wt. % | Wt. % |
| **Oil Phase** | | |
| Soybean Oil | 50% | 35% |
| BHT Food Grade Antioxidant | 0.40% | 0.28% |

| **Aqueous Phase** | | |
|---|---|---|
| Sodium Cocoyl Glutamate (Amisoft® CS-11) | 10% | 7% |
| Deionized water | Q.S.* | Q.S.* |
| DMDM Hydantoin (and) lodopropynyl Butylcarbamate( Glydant™ Plus™ Lilquid) | 0.40% | 0.40% |
| Process pressure, PSI | 5000 | 5000 |
| Pass# | 1 | 1 |
| D_{[3,2]} nm | 113 | 141 |
| D_{[4,3]j} nm | 157 | 243 |

| | | |
|---|---|---|
| * Amount needed (e.g., to obtain 100% by wt.) | | |

From Example 2 versus Example 1, it can be seen that at oil levels of 40% and above (Example 1), preferred size of triglyceride is 225 nm and below; preferably 20 to 225. Example 2 comparably shows that, at lower levels (e.g., 35% of nanoemulsion), these preferred sizes are not shown.

**Examples 3 to 6** Mono-sodium or potassium cocoyl glutamate was used as emulsifier for petrolatum nanoemulsions. Petrolatum G2212 oil droplets were reduced to 270 nm and 195 nm after passing through the Nano DeBEE at 5000 psi once and twice respectively. For white petrolatum examples, oil droplets were reduced to 374 nm or 280 nm after passing nano DeBEE once at 5000 psi once or twice respectively.

| | Example 3 | Example 4 | Example 5 | Example 6 |
|---|---|---|---|---|
| Ingredient | Wt. % | Wt. % | Wt% | Wt% |
| **Oil Phase** | | | | |
| Petrolatum G2212 | 50% | 50% | | |
| White petrolatum | | | 50% | 50% |

| **Aqueous Phase** | | | | |
|---|---|---|---|---|
| Sodium cocoyl Glutamate (AMISOFT® CS-11) | 8% | 8% | | |
| Potasium cocoyl Glutamate (AMISOFT® CK-11) | | | 8% | 8% |
| Deionized Water | Q.S. | Q.S. | Q.S. | Q.S. |
| DMDM Hydantoin (and) lodopropynyl Butylcarbamate(Glydant™ Plus™ Liquid) | 0.40% | 0.40% | 0.40% | 0.40% |
| Process pressure, PSI | 5000 | 5000 | 5000 | 5000 |
| Number of Passes | 1 | 2 | 1 | 2 |
| D_{[3,2]} nm | 162 | 133 | 188 | 165 |
| D_{[4,3]j} nm | 270 | 195 | 374 | 280 |

For petrolatum, at levels above 40% of nanoemulsion, preferred sizes of 20 to 400 nm (measured at 5000 psi pressure) are obtained.

**Examples 7 to 8** Potassium cocoyl glutamate was used as emulsifier for soybean oil nanoemulsions. Oil droplets were reduced to 188 nm and 268 nm after passing through the Nano DeBEE once at 5000 psi and 3000 psi respectively with oil level 55%.

| | Example 7 | Example 8 |
|---|---|---|
| Ingredient | Wt. % | Wt% |
| **Oil Phase** | | |
| Soybean Oil | 55% | 55% |
| BHT Food Grade Antioxidant | 0.4% | 0.4% |
| | | |

| **Aqueous Phase** | | |
|---|---|---|
| Potasium cocoyl Glutamate (AMISOFT® CK-11) | 8.8% | 8.8% |
| | | |
| Deionized Water | Q.S. | Q.S. |
| DMDM Hydantoin (and) lodopropynyl Butylcarbamate(Glydant™ Plus™ Liquid) | 0.4% | 0.4% |
| Process pressure, PSI | 5000 | 3000 |
| D_{[3,2]} nm | 127 | 163 |
| D_{[4,3]j} nm | 188 | 268 |

It is noted that, for Example 8, preferred smaller size is not obtained because pressure was 3000 psi. At 4000-5000 psi, size of 20 to 225 nm is clearly obtained.

Example 9. A personal cleanser liquid was prepared by blending the nanoemulsion according to Example 3 and the cationic polymer Jaguar@ C-13S dispersion in glycerol.

| Ingredient | Wt. % |
|---|---|
| Nanoemulsion (Example 3) | 94.6% |
| Jaguar® C-13S | 0.4% |
| Glycerol | 5% |

## Claims

1. Nanoemulsion composition comprising:
a) an internal phase comprising 40 to 75% by wt. of total nanoemulsion composition of oils selected from the group consisting of triglyceride, petrolatum and mixtures thereof, wherein the melting point of the petrolatum is 30 to 60°C; and
b) an external aqueous phase comprising 2 to 15% by wt. (as active) of total nanoemulsion composition of a surfactant or surfactants which are N-acyl derivatives of di-carboxylic amino acid salt;
wherein the surfactant of (b) comprises 50% or greater of all surfactant present in said external aqueous phase of said nanoemulsion;
wherein the volume average diameter of oil droplets of (a) is 20 to 500 nanometers.

2. A nanoemulsion composition according to claim 1, wherein said surfactant or surfactants are selected from the group consisting of
(i) salt of acylglutamic acid, wherein greater than 65% (e.g., 65 to 100%, preferably 65 to 90%) of the acyl group has chain length of C₁₄ or less; and
(ii) salt of acylaspartic acid, wherein greater than 65% of the acyl group (e.g., 65 to 100%, preferably 65 to 90%) has chain length C₁₄ or less; and
(iii) mixtures thereof

3. A nanoemulsion composition according to claims 1 or 2 wherein component (a) comprise 41 to 70% by wt. of the nanoemulsion composition and the volume average diameter of the oil droplets is 20 to 400 nanometers.

4. A nanoemulsion composition according to any of claims 1-3 wherein internal phase comprises triglycerides and volume average diameter of the droplets is 20 to 225 nm.

5. A nanoemulsion according to any of claims 1-3, wherein internal phase comprises petrolatum and volume average diameter of droplets is 20 to 400 nm, preferably 25 to 350 nm.

6. A nanoemulsion composition according to any of claims 1-4 where the oil is a triglyceride oil and said glyceride oil is selected from the group consisting of soybean oil, sunflower seed oil, coconut oil, rapeseed oil, palm oil, palm kernel oil, fish oil and mixtures thereof.

7. A nanoemulsion according to any of claims 1-3 wherein the oil is a mixture of triglyceride oil and petrolatum.

8. A nanoemulsion composition according to any of claims 1-7 wherein the salts of acylglutamate and acylaspartate are sodium and/or potassium salts.

9. A nanoemulsion composition according to any of claims 1-8 wherein the nanoemulsion is prepared at a pressure from a sonolator or other type of homogenizer and said pressure is 4.83 10⁷ Pa (7000 psi) or below.

10. A personal care composition comprising:
a) 90 to 99% by wt. of nanoemulsion of any of claims 1 to 9;
b) 0.1 to 3% cationic polymer; and
c) 0.9 to 9% dispersing agent.

11. A process for preparing a nanoemulsion comprising:
a) an internal phase comprising 40 to 75% by wt. of total nanoemulsion composition of oils selected from the group consisting of triglyceride, petrolatum and mixtures thereof, wherein the melting point of the petrolatum is 30 to 60°C; and
b) an external aqueous phase comprising 2 to 15% by wt. (as active) of total nanoemulsion composition of a surfactant or surfactants which are N-acyl derivatives of di-carboxylic amino acid salt;
wherein the surfactant of (b) comprises 40% or greater of all surfactant present in said external aqueous phase of said nanoemulsion;
wherein the volume average diameter of oil droplets of (a) is 20 to 500 nanometers;
wherein said process comprises:
1) heating aqueous phase to about 55 to about 75°C;
2) heating oil phase to about 55 to about 75°C or until oil is molten;
3) adding oil phase to aqueous phase to form a coarse emulsion with a conventional rotator/stator high shear device at speed of 1000 to 6000 revolutions per minute (rpm) ;
4) pumping the coarse emulsion once or multiple times through a homogenizer at a process pressure of 4.83 10⁷ Pa (7000 psi) or less, preferably 4.14 107 Pa (6000 psi) or less; preferably 3.45 10⁷ Pa (5000 psi) or less; and
5) cooling emulsion to room temperature.

12. A process according to claim 11 wherein, in step 3), the coarse emulsion is formed using a homogenizer operating at pressure of 1.38 10⁶ to 3.45 10⁶ Pa (200 to 500 psi)

## Patentansprüche

1. Nanoemulsionszusammensetzung, umfassend:
a) eine innere Phase, umfassend 40 bis 75 Gew.-% der gesamten Nanoemulsionszusammensetzung Öle, ausgewählt aus der Gruppe, bestehend aus Triglycerid, Petrolatum und Mischungen davon, wobei der Schmelzpunkt des Petrolatums 30 bis 60 °C beträgt; und
b) eine äußere wässrige Phase, umfassend 2 bis 15 Gew.-% (als Aktiv) der gesamten Nanoemulsionszusammensetzung eines Tensids oder an Tensiden, die N-Acyl-Derivate von Amino-Dicarbonsäuresalz sind;
wobei das Tensid von (b) 50 % oder mehr von allem Tensid, das in der äußeren wässrigen Phase der Nanoemulsion vorliegt, umfasst;
wobei der volumenmittlere Durchmesser der Öltröpfchen von (a) 20 bis 500 Nanometer ist.

2. Nanoemulsionszusammensetzung nach Anspruch 1, wobei das Tensid oder die Tenside aus der Gruppe ausgewählt sind, bestehend aus:
(i) Salz von Acylglutaminsäure, wobei mehr als 65 % (z.B. 65 bis 100 %, vorzugsweise 65 bis 90 %) der Acyl-Gruppe eine Kettenlänge von C₁₄ oder weniger aufweisen; und
(ii) Salz von Acylasparaginsäure, wobei mehr als 65 % der Acyl-Gruppe (z.B. 65 bis 100 %, vorzugsweise 65 bis 90 %) eine Kettenlänge C₁₄ oder weniger aufweisen; und
(iii) Mischungen davon.

3. Nanoemulsionszusammensetzung nach Anspruch 1 oder 2, wobei der Bestandteil (a) 41 bis 70 Gew.-% der Nanoemulsionszusammensetzung umfasst und der volumenmittleren Durchmesser der Öltröpfchen 20 bis 400 Nanometer beträgt.

4. Nanoemulsionszusammensetzung nach irgendeinem der Ansprüche 1-3, wobei die innere Phase Triglyceride umfasst und der volumenmittlere Durchmesser der Tröpfchen 20 bis 225 nm beträgt.

5. Nanoemulsionszusammensetzung nach irgendeinem der Ansprüche 1-3, wobei die innere Phase Petrolatum umfasst und der volumenmittlere Durchmesser der Tröpfchen 20 bis 400 nm, vorzugsweise 25 bis 350 nm, beträgt.

6. Nanoemulsionszusammensetzung nach irgendeinem der Ansprüche 1-4, wobei das Öl ein Triglyceridöl ist und das Glyceridöl aus der Gruppe ausgewählt ist, die aus Sojaöl, Sonnenblumenkernöl, Kokosnussöl, Rapsöl, Palmöl, Palmkernöl, Fischöl und Mischungen davon besteht.

7. Nanoemulsion nach irgendeinem der Ansprüche 1-3, wobei das Öl eine Mischung aus Triglyceridöl und Petrolatum ist.

8. Nanoemulsionszusammensetzung nach irgendeinem der Ansprüche 1-7, wobei die Salze des Acylglutamats und Acylaspartats Natrium- und/oder Kalium-Salze sind.

9. Nanoemulsionszusammensetzung nach irgendeinem der Ansprüche 1-8, wobei die Nanoemulsion unter Druck mit einem Sonolator oder einem anderen Homogenisatortyp hergestellt wird und der Druck 4,83·10⁷ Pa (7000 psi) oder darunter beträgt.

10. Körperpflegezusammensetzung, umfassend:
a) 90 bis 99 Gew.-% der Nanoemulsion nach irgendeinem der Ansprüche 1 bis 9;
b) 0,1 bis 3 % kationisches Polymer; und
c) 0,9 bis 9% Dispergiermittel.

11. Verfahren zur Herstellung einer Nanoemulsion, umfassend:
a) eine innere Phase, umfassend 40 bis 75 Gew.-% der gesamten Nanoemulsionszusammensetzung Öle, ausgewählt aus der Gruppe, bestehend aus Triglycerid, Petrolatum und Mischungen davon, wobei der Schmelzpunkt des Petrolatums 30 bis 60 °C beträgt; und
b) eine äußere wässrige Phase, umfassend 2 bis 15 Gew.-% (als Aktiv) der gesamten Nanoemulsionszusammensetzung eines Tensids oder an Tensiden, die N-Acyl-Derivate von Amino-Dicarbonsäuresalz sind;
wobei das Tensid von (b) 40 % oder mehr von allem Tensid umfasst, das in der äußeren wässrigen Phase der Nanoemulsion vorliegt;
wobei der volumenmittlere Durchmesser der Öltröpfchen von (a) 20 bis 500 Nanometer beträgt,
wobei das Verfahren umfasst:
1) Erhitzen der wässrigen Phase auf etwa 55 bis etwa 75 °C;
2) Erhitzen der Ölphase auf etwa 55 bis etwa 75 °C oder bis das Öl geschmolzen ist;
3) Zugeben von Ölphase zur wässrigen Phase, um eine grobe Emulsion mit einem konventionellen Rotor/Stator-Hochschergerät bei einer Geschwindigkeit von 1000 bis 6000 Umdrehungen pro Minute (U/min) zu bilden;
4) Pumpen der groben Emulsion einmal oder mehrmals durch einen Homogenisator bei einem Verfahrensdruck von 4,83·10⁷ Pa (7000 psi) oder weniger, vorzugsweise von 4,14·10⁷ Pa (6000 psi) oder weniger, vorzugsweise 3,45·10⁷ Pa (5000 psi) oder weniger; und
5) Kühlen der Emulsion auf Raumtemperatur.

12. Verfahren nach Anspruch 11, wobei im Schritt 3) die grobe Emulsion unter Verwendung eines Homogenisators gebildet wird, der bei einem Druck von 1,38·10⁶ bis 3,45·10⁶ Pa (200 bis 500 psi) betrieben wird.

## Revendications

1. Composition de nanoémulsion comprenant :
a) une phase interne comprenant de 40 à 75 % en masse de composition de nanoémulsion totale d'huiles choisies dans le groupe consistant en triglycéride, pétrolatum et mélanges de ceux-ci, dans laquelle le point de fusion du pétrolatum est de 30 à 60°C ; et
b) une phase aqueuse externe comprenant de 2 à 15 % en masse (comme actif) de composition de nanoémulsion totale d'un tensioactif ou de tensioactifs qui sont des dérivés N-acyliques de sel d'acide aminé di-carboxylique ;
dans laquelle le tensioactif de (b) comprend 50 % ou plus de tous les tensioactifs présents dans ladite phase aqueuse externe de ladite nanoémulsion ;
dans laquelle le diamètre moyen en volume de gouttelettes d'huile de (a) est de 20 à 500 nanomètres.

2. Composition de nanoémulsion selon la revendication 1, dans laquelle ledit tensioactif ou lesdits tensioactifs sont choisis dans le groupe consistant en
(i) sel d'acide acylglutamique, dans laquelle plus de 65 % (par exemple, de 65 à 100 %, de préférence de 65 à 90 %) du groupe acyle présentent une longueur de chaîne de C₁₄ ou inférieure ; et
(ii) sel d'acide acylaspartique, dans laquelle plus de 65 % du groupe acyle (par exemple, de 65 à 100 %, de préférence de 65 à 90 %) présentent une longueur de chaîne C₁₄ ou inférieure ; et
(iii) des mélanges de ceux-ci.

3. Composition de nanoémulsion selon les revendications 1 ou 2, dans laquelle le constituant (a) comprend de 41 à 70 % en masse de la composition de nanoémulsion et le diamètre moyen en volume des gouttelettes d'huile est de 20 à 400 nanomètres.

4. Composition de nanoémulsion selon l'une quelconque des revendications 1-3, dans laquelle la phase interne comprend des triglycérides et un diamètre moyen en volume des gouttelettes est de 20 à 225 nm.

5. Nanoémulsion selon l'une quelconque des revendications 1-3, dans laquelle la phase interne comprend du pétrolatum et le diamètre moyen en volume de gouttelettes est de 20 à 400 nm, de préférence de 25 à 350 nm.

6. Composition de nanoémulsion selon l'une quelconque des revendications 1-4 où l'huile est une huile de triglycéride et ladite huile de glycéride est choisie dans le groupe consistant en huile de soja, huile de graine de tournesol, huile de noix de coco, huile de colza, huile de palme, huile de palmiste, huile de poisson et mélanges de celles-ci.

7. Nanoémulsion selon l'une quelconque des revendications 1-3, dans laquelle l'huile est un mélange d'huile de triglycéride et de pétrolatum.

8. Composition de nanoémulsion selon l'une quelconque des revendications 1-7, dans laquelle les sels de glutamate d'acyle et d'aspartate d'acyle sont des sels de sodium et/ou de potassium.

9. Composition de nanoémulsion selon l'une quelconque des revendications 1-8, dans laquelle la nanoémulsion est préparée à une pression à partir d'un sonolateur ou autre type d'homogénéisateur et ladite pression est de 4,83 10⁷ Pa (7 000 psi) ou inférieure.

10. Composition pour le soin de la personne comprenant :
a) de 90 à 99 % en masse de nanoémulsion selon l'une quelconque des revendications 1 à 9 ;
b) de 0,1 à 3 % de polymère cationique ; et
c) de 0,9 à 9 % d'agent dispersant.

11. Procédé de préparation d'une nanoémulsion comprenant :
a) une phase interne comprenant de 40 à 75 % en masse de composition de nanoémulsion totale d'huiles choisies dans le groupe consistant en triglycéride, pétrolatum et mélanges de ceux-ci, dans laquelle le point de fusion du pétrolatum est de 30 à 60°C ; et
b) une phase aqueuse externe comprenant de 2 à 15 % en masse (comme actif) de composition de nanoémulsion totale d'un tensioactif ou de tensioactifs qui sont des dérivés N-acyliques de sel d'acide aminé di-carboxylique ;
dans lequel le tensioactif de (b) comprend 40 % ou plus de tous les tensioactifs présents dans ladite phase aqueuse externe de ladite nanoémulsion ;
dans lequel le diamètre moyen en volume de gouttelettes d'huile de (a) est de 20 à 500 nanomètres ;
dans lequel ledit procédé comprend :
1) le chauffage d'une phase aqueuse à d'environ 55 à environ 75°C ;
2) le chauffage d'une phase d'huile à d'environ 55 à environ 75°C ou jusqu'à ce que l'huile soit fondue ;
3) l'addition d'une phase d'huile à une phase aqueuse pour former une émulsion grossière avec un dispositif de cisaillement élevé à rotor/stator classique à une vitesse de 1 000 à 6 000 tours/minute (tr/min) ;
4) le pompage de l'émulsion grossière une ou plusieurs fois à travers un dispositif d'homogénéisation à une pression de procédé de 4,83 10⁷ Pa (7 000 psi) ou inférieure, de préférence 4,14 10⁷ Pa (6 000 psi) ou inférieure, de préférence 3,45 10⁷ Pa (5 000 psi) ou inférieure ; et
5) le refroidissement de l'émulsion jusqu'à température ambiante.

12. Procédé selon la revendication 11 dans lequel, dans l'étape 3), l'émulsion grossière est formée en utilisant un dispositif d'homogénéisation fonctionnant à une pression de 1,38 10⁶ à 3,45 10⁶ Pa (200 à 500 psi).
